# FASCICULE DE BREVET EUROPEEN

(11) **EP 1 946 076 B1**
(45) Date de publication et mention de la délivrance du brevet: **04.06.2014**
(21) Numéro de dépôt: 06819334.1
(22) Date de dépôt: 08.11.2006
(51) Int. Cl.: G01N 21/33, G01N 33/543, G01N 21/25

(54) **SYSTÈME D'IMAGERIE DE BIOPUCE**
BIOCHIPABBILDUNGSSYSTEM
BIOCHIP IMAGING SYSTEM

(30) Priorité: 08.11.2005 FR 0511346
(43) Date de publication de la demande: 23.07.2008
(73) Titulaire: THALES, 92200 Neuilly sur Seine (FR)
(72) Inventeur: REVERCHON, Jean-Luc, F-75005 Paris (FR); MAZZEO, Giovanni, I-00154 Rome (IT)
(74) Mandataire: Esselin, Sophie
(86) Numéro de dépôt international: PCT/EP2006/068238
(87) Numéro de publication internationale: WO 2007/054518

(56) Documents cités:
- EP-A- 1 281 969
- WO-A-02/093144

## Description

Le domaine de l'invention est celui des biopuces, telles que les puces ADN ou à protéines ou les capteurs immunologiques et des dispositifs d'imagerie associés.

Les biopuces sont un outil biochimique de collection massive d'information sur les acides nucléiques (puces ADN) et les acides aminés (puces à protéines), les antigènes et anticorps (capteurs immunologiques). Associées à des techniques de traitement numérique des informations récoltées, les puces ADN permettent de mener les recherches (détection, séparation, identification, étude) permettant d'accéder directement aux ADN. Les puces à protéines permettent de détecter, identifier, séparer, étudier les protéines et déterminer leurs activités, fonctions, interactions, modifications au cours du temps .... Les capteurs immunologiques basés sur une liaison avec une enzyme permettent de détecter des anticorps/antigènes.

D'une manière générale l'invention concerne donc des biopuces, qui se présentent sous forme d'un support solide à la surface duquel des éléments biochimiques sont immobilisés. La position de chaque spot d'éléments biochimiques est connue et éventuellement sa composition. S'agissant d'une puce ADN par exemple, les éléments biochimiques sont par exemple des oligonucléotides (simples brins) spécifiques connus. Leur rôle est de détecter des cibles marquées complémentaires, présentes dans le mélange complexe à analyser. En effet, le principe de détection utilisé dans les puces ADN repose sur les possibilités d'appariement de brins d'ADN avec leurs bases complémentaires.

On rappelle que l'ADN et l'ARN ont une forme d'hélice comprenant deux chaînes de nucléotides complémentaires et antiparallèles. Les chaînes nucléotides d'ADN, ou d'ARN sont caractérisées par quatre bases azotées, qui confèrent aux composés biologiques des propriétés capitales. Pour l'ADN, les quatre bases sont l'adénine A, la cytosine C, la guanine G et la thymine T. Pour l'ARN, l'uracile remplace la thymine, les trois autres bases étant communes à l'ADN. Ces bases sont complémentaires deux à deux : A s'apparie avec T ou U et G avec C. C'est cette capacité pour des monobrins d'ADN, ou ADN monocaténaire, de former une double hélice par appariement des bases complémentaires qui est utilisée dans le principe de détection par puce ADN. C'est l'hybridation : les sondes de la biopuce sont mises en contact avec l'échantillon à analyser. Si l'échantillon comporte la séquence complémentaire d'une sonde, il vient s'accrocher sur cette sonde. On obtient une puce hybridée. II s'agit alors de détecter et de quantifier l'ensemble des cibles recherchées dans le mélange à analyser, par analyse de la puce hybridée. Les puces à protéines et les capteurs immunologiques fonctionnent sur un principe similaire, pour la détection de chaînes d'acides aminés pour les premières, et d'anticorps/antigènes pour les deuxièmes.

En pratique, une biopuce comporte un très grand nombre de spots selon une disposition matricielle ordonnée. Les sondes sont soit greffées sur le support, soit synthétisées in situ (unité d'hybridation = spot). La haute densité de spots permet la détection en parallèle de milliers de séquences. La technologie des biopuces permet ainsi de couvrir le génome (puces ADN) ou le protéome (puce à protéine) d'un organisme avec une unique puce.

Le support solide est typiquement une lamé de verre de taille comparable à une lame de microscope. On trouve aussi des supports silicium ou nylon. Un spot est l'unité d'hybridation de la puce. Si on prend l'exemple d'une puce à ADN, des fragments d'ADN qui constituent une sonde spécifique sont déposés sur chaque spot de cette puce. Dans un exemple, on peut avoir des spots de l'ordre de 100µm et 1000 à 10000 spots par cm². Ce sont autant de sondes qui permettent d'hybrider des séquences d'ADN complémentaires provenant d'un échantillon biologique à analyser. La position et la composition de la séquence de chaque sonde (sur chaque spot) est connue. On dit que la puce est fonctionnalisée pour détecter des cibles particulières.

En pratique, on vient mettre en contact cette sonde avec le mélange complexe à analyser, qui peut se présenter en solution ou sur un substrat solide, de manière à permettre l'appariement par base complémentaire. On vient ensuite laver la puce, pour éliminer ce qui ne s'est pas apparié. On obtient une puce hybridée qu'il faut analyser.

Des dispositifs d'imagerie sont utilisés à cet effet, qui permettent de réaliser une cartographie de la biopuce.

Selon l'état de l'art, on utilise généralement des marqueurs, de type radioactifs ou fluorescents qui sont ajoutés aux échantillons, pour leur conférer des propriétés émettrices qui sont alors utilisées pour la détection des chaînes, par mesure du signal correspondant, radioémissif ou fluorescent. Les images obtenues sont numérisées pour être ensuite traitées par des algorithmes de traitement spécifiques de ces données, mis en oeuvre par des moyens informatiques.

Un marquage couramment employé est le marquage physico-chimique, par des marqueurs fluorescents, c'est à dire des composés chromophores. Ces systèmes sont très utilisés car ils offrent une bonne sensibilité de détection.

Selon le protocole de marquage physico-chimique, les brins des échantillons sont marqués par des composés chromophores, le plus souvent, du cyanine 3 ou 5. On peut alors procéder par comparaison du marquage d'une cible inconnue avec celui d'une cible de référence en utilisant un marqueur différent pour chacune d'entre elles. Pour générer une image complète de la puce hybridée, il est nécessaire d'acquérir une image correspondant à chaque composé chromophore. On mesure l'intensité du signal fluorescent émis sur les différents spots, dans chaque bande spectrale. Ceci peut se faire au moyen d'un scanner par exemple. Il existe dans le commerce des scanners adaptés à cette utilisation. Pour mémoire, le principe de détection est alors le suivant : la puce est placée en chambre noire. Le scanner éclaire la lame au moyen d'un faisceau laser de grande puissance. Par effet de fluorescence, on obtient l'émission d'un faisceau dans une bande spectrale différente, fonction du marqueur, à l'endroit des différents spots sur lesquels des brins se sont appariés, faisceau qui est détecté par le scanner. Une analyse des images permet d'extraire les signaux d'hybridation de chacune des sondes. Les images sont obtenues sous forme numérique. Des traitements de données de ces images permettent alors d'identifier, et de quantifier les analytes dans le mélange analysé, de comparer les résultats obtenus à ceux d'échantillons connus. Les mesures des signaux peuvent être affinées par des techniques d'élimination du bruit de fond, notamment l'auto-fluorescence du support. Cette technique selon l'état de l'art offre une très bonne sensibilité de détection, nécessaire à toute la palette des applications des puces ADN.

Ainsi, la technique d'hybridation des puces ADN est-elle combinée étroitement à la technique de marquage fluorescent ou radio-actif, au point d'en être indissociable, ce qui ressort de nombreuses publications.

On trouve ainsi dans le glossaire bioinfo-biotechnologies, accédé à la date du 27 octobre 2005 depuis la page d'accueil Infobiogen © du centre National de Ressources Informatiques Appliquées à la Génétique (http://www.infobiogen.fr), la définition suivante pour "Puce à ADN - Biopuce - matrice" : "*Technique d'hybridation permettant une analyse génomique comparative de l'expression d'un grand nombre de patterns d'ARNm. Immobilisés sur un support solide (matrice), des oligonucléotides (simples brins) spécifiques de différents gènes ou ADNc connus constituent les sondes dont le rôle est de détecter des cibles marquées complémentaires, présentes dans le mélange complexe à analyser (ARNm extraits de cellules, tissus ou organismes entiers et convertis en ADNc). Les sondes sont soit greffées sur le support, soit synthétisées in situ (unité d'hybridation = plot). Les signaux d'hybridation sont détectés selon le type de marquage, radioactivité ou fluorescence, par mesure radiographique ou par fluorescence, et quantifiés".* On trouve encore à cette même date sur le site de l'Institut Pasteur (http://www.pasteur.fr) dans un dossier intitulé "La génomique à l'Institut Pasteur" que s'agissant des biopuces, *"Ces outils, dont le concept date de la fin des années 80, servent à rechercher simultanément la présence de milliers de gènes (ou de produits de gènes (ARNm)) donnés dans une solution contenant des séquences d'ADN (ou d'ARN) inconnues. Pour cela, des milliers d'* " *hameçons* " *moléculaires (sondes) différents, chacun spécifique d'un gène (ou d'un ARNm) recherché sont fixés sur un support. Ce support est mis au contact d'une solution contenant les séquences à analyser, marquées par fluorescence. Si un gène (ou un ARNm) est au contact d'une sonde qui lui est spécifique, il s'y fixera. Dans le cas contraire, il restera " libre* " *et sera évacué par rinçage. Au final, des spots fluorescents sur le support indiqueront quelles sont les sondes ayant fixé leur gène (ou ARNm) spécifique, et donc quels gènes (ou ARNm) recherchés étaient présents dans l'échantillon analysé."*

Cette technique a pourtant de nombreux inconvénients. Elle rend plus complexe le procédé d'hybridation de la puce, imposant des étapes supplémentaires, coûteuses et sensibles pour réaliser le marquage. Les chromophores utilisés comme marqueurs sont des composés coûteux, et ont une faible durée de vie. Ils introduisent des biais d'incorporation et de lecture, dus à la présence de plusieurs marqueurs par spots, ou dus à la détérioration des propriétés physico-chimiques des brins du fait des marqueurs. Ils présentent des phénomènes de vieillissement, notamment sous l'effet de l'illumination.

On connait par ailleurs de la demande EP1281969, un système de détection de l'autofluorescence dans la bande spectrale UV, d'une protéine disposée sur une biopuce et excitée par un faisceau de lumière UV et fluorescente à longueur d'onde supérieure à la longueur d'excitation.

Dans l'invention, on a cherché un dispositif de détection directe, c'est à dire un dispositif qui ne nécessite pas l'utilisation de marqueurs, mais qui utilise les propriétés optiques intrinsèques des éléments détectés, c'est à dire les caractéristiques d'absorption des rayonnements ultra-violets. Selon le type de biopuce, des longueurs d'onde peuvent être plus particulièrement intéressantes. A titre d'exemple, pour les puces à ADN on s'intéresse plus particulièrement aux caractéristiques d'absorption à 260nm ; à 280nm pour les puces à protéines.

Les techniques d'électrophorèse, notamment l'électrophorèse capillaire utilisent ces propriétés optiques, notamment pour doser la pureté des acides nucléiques. L'électrophorèse capillaire utilise un capillaire de silice fondue, qui a comme propriété d'être transparent à 260 nanomètres, les deux extrémités du capillaire formant une anode et une cathode. Le capillaire est rempli d'un gel d'électrophorèse dans lequel est injecté l'échantillon à analyser, à l'extrémité de la cathode. Sous l'effet d'un champ électrique obtenu par application d'une tension électrique appliquée entre anode et cathode, on obtient une migration des molécules vers l'anode. La mobilité efficace des molécules décroît quand le rapport charge/masse décroît. Sur le plan électrique, le support (gel) n'intervient pas. Par contre sa porosité conditionne la vitesse de migration des molécules selon leur taille, à charge égale. Cette propriété permet de déterminer la taille des fragments d'ADN contenus dans un mélange à analyser, après calibrage d'une échelle de taille pour un support d'électrophorèse déterminé, au moyen de fragments de taille connue. Un système d'imagerie associé comprenant par exemple un capteur CCD permet de mesurer l'absorption UV par les différentes molécules directement sur le capillaire, selon un système de détection transmissif : d'un côté du capillaire, la source de rayonnement ultraviolet ; de l'autre, un capteur CCD qui récupère l'image du contraste d'absorption.

Les techniques d'électrophorèse et les techniques associées aux biopuces ne sont pas des techniques qui couvrent les mêmes applications, en sorte qu'elles ne sont pas équivalentes. En outre, les techniques d'électrophorèse ont aussi recours à des techniques de marquage, par exemple fluorescent, ou à des techniques d'amplification, par exemple quand il s'agit de distinguer des fragments de même taille, qui ont la même migration dans le capillaire.

Dans l'invention, on a cherché à utiliser les propriétés optiques d'absorption aux UV pour la détection des cibles sur les biopuces hybridées.

Le problème lié à l'utilisation de ces propriétés optiques d'absorption dans l'ultra-violet est principalement lié à la possibilité d'obtenir un contraste suffisant, par rapport au support notamment.

Dans le contexte de l'électrophorèse, le chemin optique du rayonnement ultra-violet est suffisamment important pour obtenir un contraste d'absorption suffisant, permettant la détection avec une bonne sensibilité. De plus, dans ces techniques, ce sont des fragments d'ADN beaucoup plus longs. Dans les biopuces, on a des chaînes d'acides nucléotides qui peuvent comporter moins de 100 bases. En électrophorèse, ce nombre varie d'une centaine à plusieurs centaines de milliers. Le capillaire a des dimensions comprises généralement entre 50 et 100 micromètres pour le diamètre interne. Le trajet optique à travers le capillaire dans les fragments d'ADN est augmenté par l'utilisation d'artifices tels que des cellules en Z ou en bulles, jusqu'à quelques millimètres.

Un objet de l'invention est de permettre l'utilisation de la détection par contraste d'absorption UV dans la puce ADN, et plus généralement dans une biopuce, pour obtenir un système d'imagerie par détection directe, qui soit suffisamment sensible mais moins coûteux que les systèmes utilisant les techniques de marquage.

La sensibilité de la détection est liée en pratique à la possibilité de révéler un contraste. Il faut considérer le support de la biopuce d'une part et les dimensions des éléments biochimiques (de la sonde, ou de la sonde hybridée avec une cible) qui sont immobilisés sur les spots d'autre part.

Le support est à l'origine d'un bruit de fond de détection. Et les éléments biochimiques sur la biopuce sont courts : ils peuvent comporter moins de 100 bases, et plus généralement moins de 5000 bases. Ils sont fixés sur le support en fines couches d'épaisseur inférieure à la centaine de nanomètres. On est bien loin des trajets optiques de quelques millimètres obtenus en électrophorèse qui permettent de révéler un contraste suffisant.

Le demandeur a cependant pu mettre en évidence, que contre toute attente, et malgré les faibles dimensions des éléments biochimiques à détecter sur les puces, une bonne sensibilité est obtenue avec un système de détection directe, qui utilise une source de lumière et/ou un détecteur spécifique à la gamme spectrale du rayonnement à détecter, combiné à un support permettant d'exacerber le contraste. De cette façon toute étape supplémentaire liée au marquage est supprimée. Les coûts associés sont supprimés.

L'invention concerne donc un système d'imagerie d'éléments biochimiques tel que défini dans la revendication 1 comprenant une biopuce comprenant un support sensiblement plan, présentant sur une face supérieure, une pluralité de spots, sur lesquels sont disposés des éléments biochimiques à analyser, une source pour éclairer ladite face supérieure de la biopuce avec un faisceau lumineux et un dispositif de détection d'un rayonnement émis par la dite face supérieure, caractérisé en ce que le dispositif de détection est agencé pour détecter le faisceau réfléchi ou transmis par ladite face supérieure du support de la biopuce, en ce que la source et le dispositif de détection opèrent chacun dans une bande de longueurs d'ondes comprenant une bande spectrale couvrant au moins une longueur d'onde d'intérêt déterminée comprise dans la bande de rayonnement ultra-violet, en ce que l'un des deux au moins de la source et du dispositif de détection est sélectif dans ladite bande spectrale et en ce que le support de ladite biopuce présente un coefficient de réflectivité ou de transmission dans ladite bande spectrale permettant un fonctionnement du système en réflexion ou en transmission.

Avantageusement le détecteur de rayonnement sélectif est un dispositif semi-conducteur à réponse sélective à la longueur d'onde d'intérêt.

Il peut s'agir d'une couche active donnant une réponse à partir de la longueur d'intérêt et éclairée à travers une couche fenêtre filtrant les longueurs d'ondes plus faibles. Un tel détecteur permet alors l'utilisation d'une source blanche du commerce comme source d'éclairage de la biopuce.

Selon un perfectionnement de l'invention, la face supérieure de la biopuce comporte des motifs sur lesquels sont disposés les spots, lesdits motifs étant tels que le chemin optique du faisceau lumineux à travers les éléments biochimiques est augmenté.

Une biopuce ayant cette caractéristique permet d'améliorer la réponse du système d'imagerie.

Selon un premier mode de réalisation, ces motifs sont des motifs géométriques protubérants, en sorte que la densité d'éléments biochimiques sur chaque spot est augmentée.

Selon un deuxième mode de réalisation, ces motifs sont des porosités, le support comprenant au moins trois couches, une première couche poreuse, dont des porosités forment les spots sur lesquels sont immobilisés des éléments biochimiques, et une couche de part et d'autre, l'une au moins également poreuse, lesdites couches de part et d'autre présentant une face réfléchissante vers ladite première couche poreuse, en sorte que le chemin optique est augmenté par effet de réflexion dans cette couche poreuse.

D'autres avantages et caractéristiques de l'invention apparaîtront plus clairement à la lecture de la description qui suit, faite à titre indicatif et non limitatif de l'invention et en référence aux dessins annexés, dans lesquels :
- la figure 1 illustre un premier exemple de réalisation d'un dispositif d'imagerie par détection directe d'un contraste d'absorption selon l'invention;
- la figure 2 illustre un deuxième exemple de réalisation d'un dispositif d'imagerie selon l'invention permettant une analyse multi-spectrale ;
- la figure 3 illustre un autre exemple de réalisation d'un dispositif d'imagerie selon l'invention, fonctionnant sur le mode transmissif ;
- la figure 4 illustre les propriétés spectrales d'un support de puces utilisé dans un dispositif d'imagerie selon l'invention,
- la figure 5 illustre les réponses spectrales d'un détecteur avec couche active sur couche fenêtre filtrante;
- la figure 6 est un schéma d'une mise en oeuvre d'un dispositif d'imagerie selon l'invention avec filtrage spectral de la source à l'aide d'un spectroscope;
- les figures 7a à 7f montrent des images obtenues avec un dispositif d'imagerie selon l'invention, utilisé avec une puce ADN;
- les figures 8a à 8d illustrent des exemples de réalisation d'un support de puce perfectionné, permettant d'améliorer la sensibilité d'un dispositif d'imagerie selon l'invention;
- les figures 9 et 10 illustrent une variante d'un système d'imagerie selon l'invention permettant d'exploiter la polarisation de la lumière;
- la figure 11 illustre un support de lame avec des réseaux favorisant une polarisation;
- la figure 12 illustre une variante des systèmes des figures 9 et 10; et
- la figure 13 illustre les différentes états de polarisation de la lumière avant et après réflexions sur une surface.

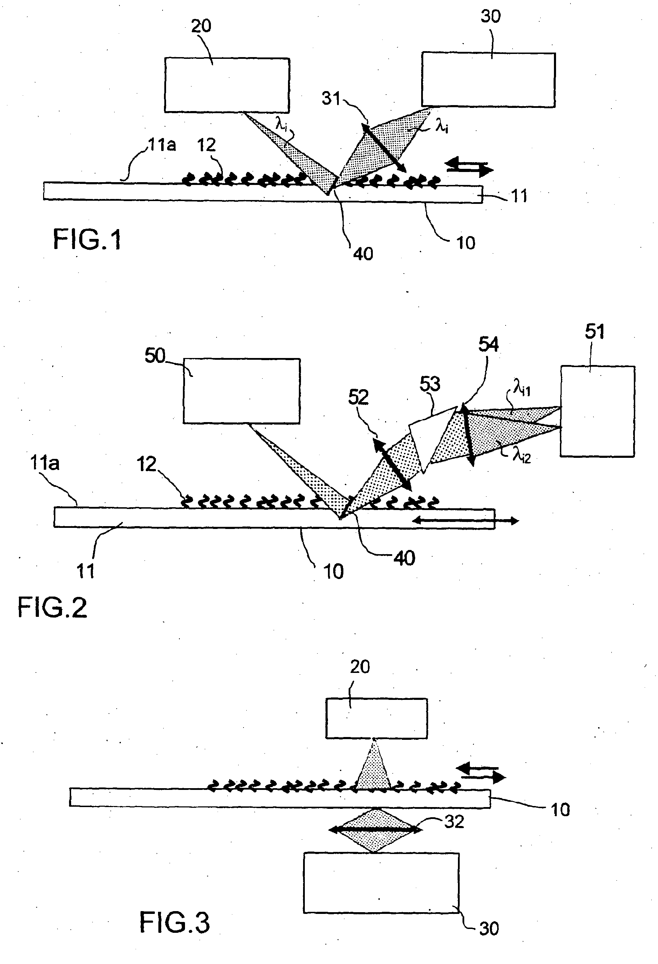

Un dispositif d'imagerie par détection directe d'un contraste d'absorption, selon l'invention comprend, comme illustré sur la figure 1 :
- une biopuce 10, comprenant principalement un support planaire 11, typiquement une lame, comportant sur sa face supérieure 11 a une pluralité de spots sur lesquels sont immobilisés des éléments biochimiques 12. La lame d'une puce à ADN a en pratique une longueur d'une dizaine de centimètres sur une largeur de quelques centimètres.
- une source d'éclairage 20 fournissant un faisceau lumineux à une longueur d'onde d'intérêt λᵢ, avec une optique de focalisation de ce faisceau sur la face supérieure de la biopuce, selon un axe 40 de focalisation.
- un dispositif de détection semi-conducteur 30 sensible à au moins la longueur d'onde d'intérêt λᵢ et une optique de focalisation 31 du rayonnement réfléchi par la lame sur la surface sensible du détecteur. Il permet de recueillir une image de l'absorption par la puce à la longueur d'onde d'intérêt, par déplacement latéral relatif de la biopuce par rapport à l'axe 40 de focalisation du rayonnement optique de façon à balayer la surface de la biopuce.

Le dispositif de détection peut comporter une simple barrette de transducteurs (photodiodes). C'est suffisant pour recueillir toute l'image d'une puce à ADN, par balayage, compte-tenu de ses dimensions habituelles, de l'ordre de quelques centimètres carrés. Il peut aussi comporter une matrice (à deux dimensions) de transducteurs. L'intérêt d'utiliser une matrice avec balayage est de limiter les effets de pixels défectueux, de bénéficier des traitements statistiques sur plusieurs colonnes (binning) et de réaliser une image hyper-spectrale si une dispersion optique est utilisée. Une matrice peut également permettre d'imager une puce sans déplacement mais avec une résolution limitée par le nombre de pixels de celle-ci.

La longueur d'onde d'intérêt λᵢ est en pratique 260 nanomètres, pour les puces ADN, correspondant au maxima d'absorption des bases azotées. Pour les puces à protéines, la longueur d'onde d'intérêt λᵢ est 280 nanomètres. Il faut que l'un au moins des dispositifs d'éclairage et de détection soit sélectif autour de la longueur d'onde d'intérêt de la biopuce, soit intrinsèquement, ou au moyen d'un filtre très sélectif. On peut ainsi utiliser une source d'éclairage ultra-violet sélective à 260 nanomètres, qui est le maxima d'absorption des bases azotées. La source sera alors typiquement une source laser, ou une diode électroluminescente, éventuellement associée à un filtre. On peut aussi utiliser une source blanche (lampe à Xénon ou au Deutérium) associée à un monochromateur.

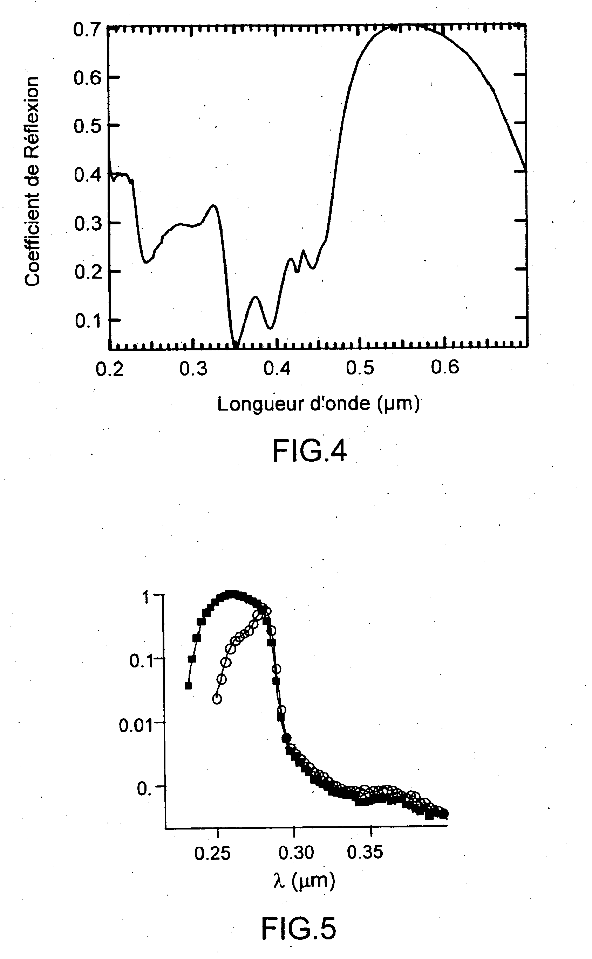

Le dispositif détection 30 est de préférence un détecteur semi-conducteur à bande spectrale étroite autour de la longueur d'onde d'intérêt λᵢ. Un tel détecteur comprend une couche active sur une couche fenêtre filtrante. Un filtrage est naturellement réalisé par la couche fenêtre pour les longueurs d'onde inférieures à 260nm et par la bande interdite de la couche active pour les longueurs d'onde supérieures. Ces détecteurs offrent ainsi une forte réponse dans la bande 260-290 nanomètres des ultra-violets tout en rejetant de plusieurs ordres de grandeur leur réponse de part et d'autre de cette bande. En particulier les détecteurs à bandgap AlGaN sensibles dans la bande 260-280 nanomètres sont particulièrement adaptés à un dispositif d'imagerie selon l'invention. La réponse d'un tel détecteur est illustrée par la courbe de la figure 5. La courbe reliant les points rectangles noirs, représente la réponse obtenue avec un éclairage par la face avant ("*front side illumination*") et celle reliant les cercles représente la réponse avec un éclairage par la face arrière à travers la couche fenêtre ("*back side illumination*"). En ordonnée, on a la réponse du détecteur et en abscisse, les longueurs d'onde en micromètres.

Une configuration avantageuse comprend alors une source blanche pour éclairer la biopuce et un détecteur à bande étroite éclairé par la face arrière, à travers la couche fenêtre. De cette façon, on n'a pas besoin de filtre propre à la source de lumière, car le détecteur travaille dans une bande spectrale étroite. Cette solution est avantageuse car les sources UV puissantes et les filtres passe-bande sont coûteux et le rejet des longueurs d'onde indésirables se fait au détriment de la transmission à la longueur d'onde d'intérêt. En utilisant un détecteur à bandgap AlGaN, on peut utiliser dans le dispositif de détection une source d'éclairage large bande du commerce, sans filtre, tout en bénéficiant d'une grande sensibilité de détection.

Dans une variante, illustrée sur la figure 2, le dispositif d'imagerie utilise plusieurs longueurs d'onde d'intérêt, dans l'exemple illustré λᵢ et λⱼ à des fins de fonctions de dispersion spectrale, autour du maxima d'absorption. De telles fonctions sont intéressantes, notamment dans le cas des puces à protéines à des fins d'identification spectrale. Le dispositif de détection sera alors une matrice si l'on veut mesurer simultanément les contrastes d'absorption à différentes longueurs d'onde. L'usage d'une barrette nécessiterait de repositionner le dispositif optique pour chaque longueur d'onde étudiée.

Le dispositif d'imagerie comprend alors une source d'éclairage 50 et un dispositif de détection 51, couvrant une plage spectrale comprenant au moins les longueurs d'onde d'intérêt.

Dans un exemple pratique, le spectre d'émission de la source 50 correspond à la bande UV B 240-290 nanomètres, et le détecteur a une sensibilité spectrale dans cette même plage.

Le dispositif d'imagerie est alors conçu pour mesurer la réponse de la puce 10 à au moins deux longueurs d'onde d'intérêt situées dans la bande B des ultra-violets, typiquement 240-290 nanomètres. Ceci s'applique aussi bien aux puces ADN qu'aux puces à protéine. On prévoit alors une source d'éclairage et un dispositif de détection correspondants. Ainsi comme illustré sur la figure 2, la source d'éclairage aura au moins un spectre d'émission correspondant, c'est à dire dans la bande UV B 240-290 nanomètres, et un détecteur sensible dans cette plage.

La source d'éclairage 50 sera alors de préférence une source de rayonnement large bande, ou blanche, typiquement une lampe au Xénon ou au Deutérium.

Le dispositif de détection 51 peut être un scanner classique du commerce, c'est à dire un détecteur de rayonnement ultra-violet en bande B [240-290] nanomètres.

De préférence, le dispositif de détection 51 est un détecteur semi-conducteur à bande spectrale étroite, notamment du type AlGaN, de grande sensibilité dans le domaine de réponse spectrale des biopuces, et dont a vu plus haut, qu'il permet lorsqu'il est éclairé par sa face arrière, de travailler dans une bande spectrale étroite même lorsque la biopuce est éclairée au moyen d'une source blanche du commerce.

Le dispositif d'imagerie illustré à la figure 2 comprend alors côté détection : une optique de collimation 52 ; une optique de dispersion 53 ; et une optique de focalisation 54 du faisceau réfléchi permettant de collecter les images différenciées en longueur d'onde.

L'optique de collimation 52 est de préférence réflective pour ne pas pâtir de la dispersion d'indice présente de 240 à 290nm. Dans un dispositif d'imagerie à bas coût, elle sera réalisée plus simplement par des lentilles. L'optique de dispersion 53 comprend par exemple un prisme, ou un réseau de diffraction. L'optique de collimation 52 et l'optique de focalisation 54 sont chacune réalisée de manière classique par une lentille. Tous ces éléments d'optiques sont bien connus de l'homme de l'art de la détection de faisceaux lumineux.

Les deux dispositifs représentés sur les figures 1 et 2 fonctionnent en réflexion. La surface 11 du support sur laquelle sont disposés les spots, présente un coefficient de réflexion le plus fort possible. Quelques dizaines de pour cent seulement sont nécessaires. L'angle d'incidence du faisceau émis par la source sur la surface 11 est par exemple de l'ordre de 45 degrés.

Le dispositif peut fonctionner en transmission. Une variante "transmissive" du dispositif réflectif de la figure 1 est illustrée sur la figure 3. Le support doit alors avoir un coefficient de transmission suffisant de l'ordre de 10 pour cent au moins. Il peut s'agir de silice fondue, de CaF₂, de saphir, de PDMS *(Polydimethyl siloxane)* jusqu'à 260nm. Il est alors possible de placer le détecteur 31 en contact direct avec l'arrière du support. Cependant l'utilisation d'une optique de focalisation 32 permet d'optimiser la collection du flux transmis et de préserver la propreté et la surface du détecteur.

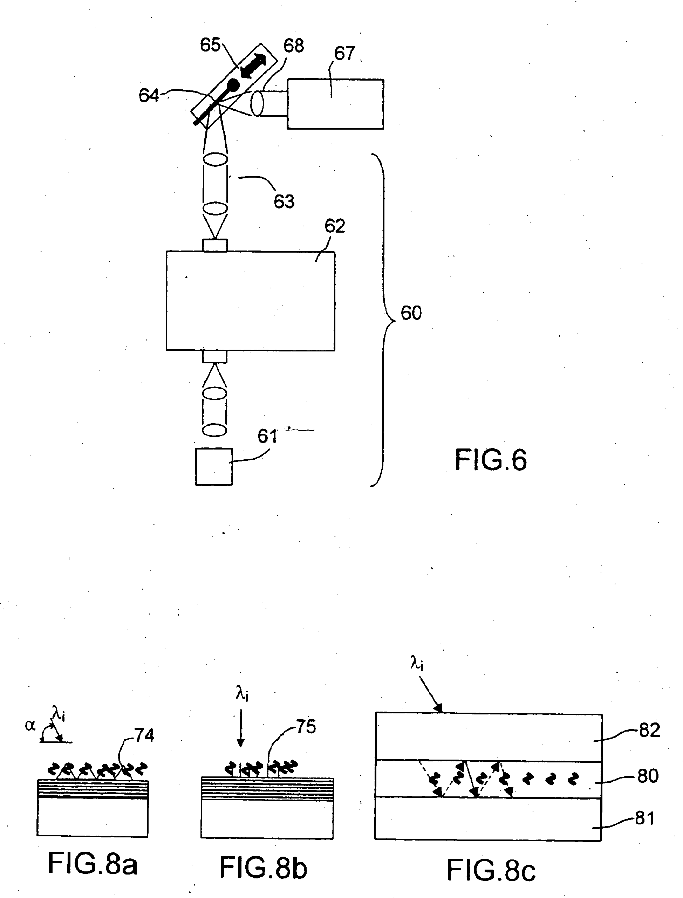

Un dispositif d'imagerie selon l'invention a été réalisé, dont la configuration est détaillée sur la figure 6. La source 60 comporte une lampe à Xénon 61 suivi d'un monochromateur 62 qui ne laisse passer en sortie que la longueur d'onde d'intérêt λᵢ (ou une fenêtre comprenant les longueurs d'onde d'intérêt), et de l'optique de focalisation 63.

La biopuce 65 est montée sur un élément permettant sa translation dans le plan, relativement à un axe 64 de focalisation de la source 60. On peut ainsi balayer l'ensemble de la lame sur toute sa longueur par le faisceau démission.

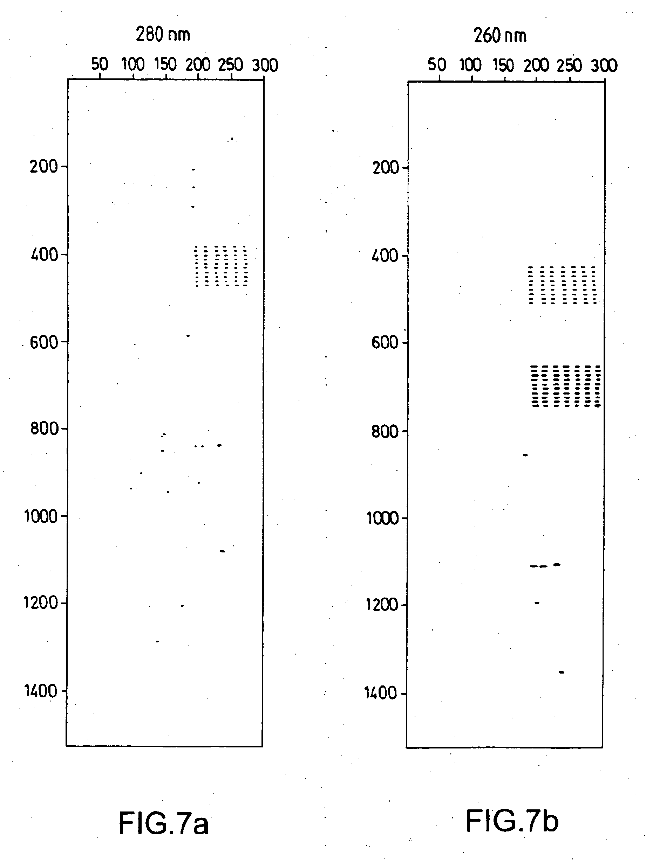

Le détecteur semi-conducteur 67, est de type AlGaN à bande spectrale étroite, comprenant une barrette de plusieurs centaines de pixels, et comprend une optique de focalisation 68 associée, c'est à dire une lentille. Dans l'expérience la biopuce est une puce ADN dont le support est une lame de verre dont la face supérieure présente un coefficient de réflexion de 30% à 260 nanomètres. Les brins d'ADN immobilisés sur les spots ont de 500 bases à 5000 bases de long. Les spots ont une taille de l'ordre de 300 micromètres. La lentille 68 réalisant l'image sur la barrette permet de réaliser une image sans agrandissement. Pour une barrette de 300 pixels et 26 micromètres de large, la largeur de la zone scannée sur la lame est de 8 millimètres. Chaque spot est ainsi susceptible d'apparaître sur une dizaine de pixels avec une résolution satisfaisante. Les images illustrées sur les figures 7a et 7b, ont été réalisées avec des illuminations à 260 nanomètres et 280 nanomètres, sur une biopuce à oligonucléotides, dont les brins ont une longueur de 70 bases. Dans ces images noir et blanc, la gamme de couleur correspond à un contraste variant de 0.95 à 1.05. Les spots sont observables aux deux longueurs d'onde mais le contraste est bien meilleur à 260 nanomètres. En particulier, pour les spots offrant le plus fort contraste, l'absorption à λᵢ=280 nm s'élève à 3% et l'absorption à λᵢ=260 nm s'élève à 5%.

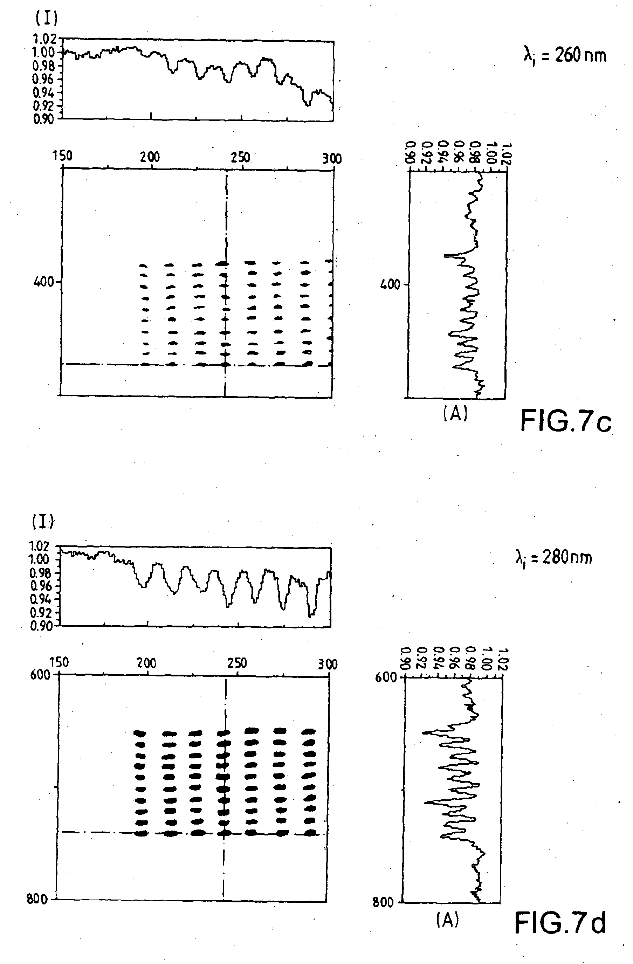

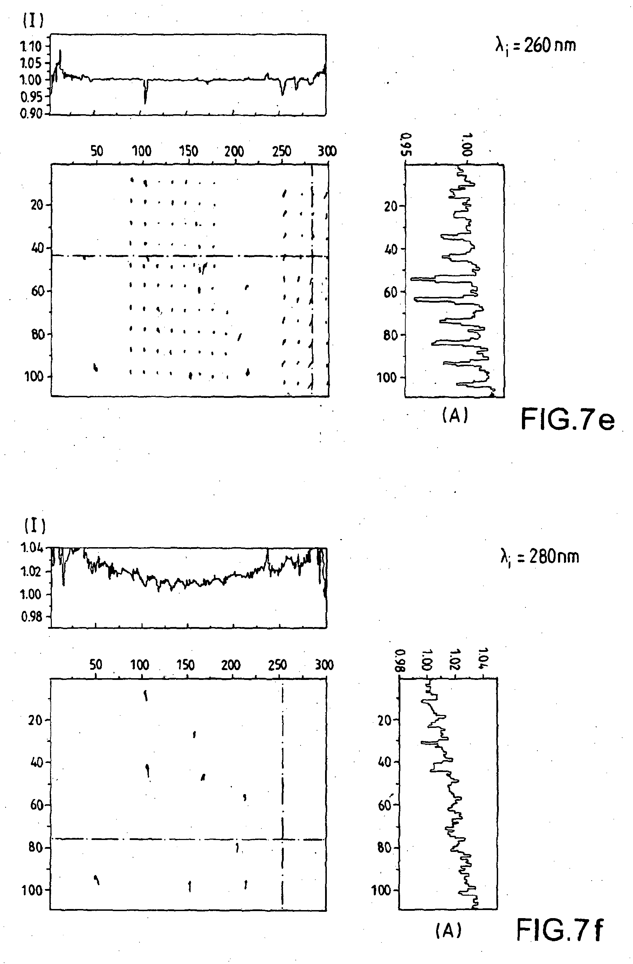

Les images illustrées sur les figures 7c et 7d, résultent de l'analyse des spots de plus forts contrastes sur les images des figures 7a et 7b. Elles montrent que l'absorption à 260nm (figure 7d) et 280 nm (figure 7c) peut être évaluée à 5 et 2 pour cent. Les images sur les figures 7e et 7f sont des images réalisées avec une biopuce, avec des simples brins d'ADN longs de 500 à 5000 bases. La première image (figure 7e) correspond à une illumination à λᵢ=260 nm, et la deuxième image (figure 7f), correspond à une illumination à λᵢ=280 nm. La gamme du contraste varie sur ces images de 0.94 à 1.1. On observe une absorption des plots de l'ordre de 4 à 5 % à 260nm.

Dans ces images, la biopuce ADN est présentée en sorte que la longueur est donnée par la verticale et la largeur par l'horizontale. Par convention, les spots sont disposés en lignes (horizontales) et en colonnes (verticales). La lame est balayée par la source d'éclairage de haut en bas. Le profil d'intensité le long d'une ligne horizontale h tracée sur l'image est illustré sur les figures 7e et 7f sous la référence I. Le profil d'absorption le long d'une colonne de spots est donnée sur les images sous la référence A.

Ces images confirment que le contraste obtenu a bien comme origine l'absorption intrinsèque des bases des éléments biochimiques, et non des effets de rugosité parasite comme par exemple des poussières. A 280 nanomètres, l'absorption des spots est quasiment masquée par le bruit à cette longueur d'onde, et ne dépasse pas 1%. En pratique, en prenant l'image obtenue avec différentes illuminations allant de 260 à 280 nm, on a pu vérifier que la poussière apparaît sur toutes les images avec le même contraste d'absorption.

La figure 4 illustre les propriétés optiques d'un support de biopuce de type réflectif utilisé dans l'invention. Ce support comporte un substrat sur lequel est réalisée une structure multicouche de matériaux, en sorte de former un miroir ayant un coefficient de réflexion non négligeable dans la gamme de longueurs d'onde autour de la longueur d'onde d'intérêt, soit typiquement autour de 260 nanomètres dans le cas des biopuces de type puces ADN, et 280 nanomètres dans le cas de biopuces de type puces à protéines.

Dans un exemple, le coefficient de réflexion peut être amélioré au moyen d'un multicouche diélectrique (Oxyde de titane et d'aluminium) ou d'un dépôt métallique. La réflectivité peut être ajustée en fonction de la longueur d'onde d'intérêt (260 ou 280 nm) en réglant les épaisseurs et la nature du multicouche diélectrique recouvrant le support. Un support comportant une couche métallique de forte réflexion à 260nm, recouverte d'une couche diélectrique de protection, sur laquelle sont disposés les spots convient à l'application peut s'avérer plus économique.

Dans un perfectionnement de l'invention, un système d'imagerie selon l'invention utilise une biopuce dont la face supérieure 11 présente une texture telle que le signal d'absorption généré par les éléments biochimiques immobilisés sur les spots se trouve amplifié. La réponse du système est améliorée.

Selon un premier mode de réalisation d'une biopuce perfectionnée, particulièrement bien adaptée à un système d'imagerie selon l'invention, on développe une forte rugosité de la surface de la biopuce quitte à la rendre légèrement poreuse à l'échelle de quelques dizaines à quelques centaines de nanomètres. On augmente ainsi la surface de greffage effective. La densité des éléments biochimiques immobilisés sur chaque spot est augmentée. On amplifie ainsi l'absorption.

Selon un autre mode de réalisation illustré sur les figures 8a et 8b, la face supérieure comporte des motifs géométriques protubérants. Ils ont pour effet d'augmenter la densité d'éléments biochimiques immobilisés sur chaque spot. Si on reprend l'exemple de la puce ADN, on augmente par cet artifice, le nombre de brins sur chaque spot. Cela revient à augmenter l'épaisseur de l'élément biochimique, et donc à augmenter le chemin optique parcouru par le faisceau incident. On amplifie ainsi l'absorption.

La face supérieure du support comprend ainsi des motifs sur lesquels sont disposés les spots. Ces motifs sont des motifs géométriques protubérants, présentant au moins un plan incliné 74 (figure 8a) ou vertical 75 (figure 8b) par rapport à la surface du support. Les éléments chimiques étant immobilisés sur ce plan. L'angle d'incidence α de l'illumination avec la surface du support est choisi pour correspondre à l'angle que forme le plan supportant les spots avec la surface, par exemple 45 degrés, pour les plans inclinés 74, 90 degrés pour les plans verticaux 75 . De cette façon, la densité d'éléments chimiques sur chaque spot est augmentée et l'angle d'incidence de l'illumination est choisi pour atteindre tous ces éléments chimiques. Le signal d'absorption de chaque spot se trouve augmenté.

Dans un autre mode de réalisation illustré sur la figure 8c, ces motifs sont tels qu'ils favorisent des réflexions du faisceau incident, en sorte que le signal d'absorption se trouve amplifié.

Dans l'exemple illustré, le support comporte une couche poreuse 80 vis à vis des éléments biochimiques à immobiliser sur les spots. Par exemple, il s'agit d'une couche de silice fondue, partiellement attaquée à l'acide, en sorte de former des porosités (des puits), Un spot peut recouvrir une large surface poreuse. Les éléments biochimiques sont donc immobilisés dans cette couche. Deux couches 81 et 82 sont situées de part et d'autre de cette couche, l'une au moins étant également poreuse. Dans l'exemple la couche 82 disposée sur la couche 80 est poreuse. Elle laisse passer les éléments biochimiques des échantillons à analyser. Ces couches 81 et 82 de part et d'autre présentent une face réfléchissante vers la première couche poreuse 80.

Le faisceau d'éclairage traverse ainsi les couches 82 et 80, et dans la couche 81 subit de multiples réflexions dues aux parois réfléchissantes des couches 82 et 81. Le chemin optique est ainsi augmenté par effet de réflexion dans la couche 80, amplifiant ainsi le signal d'absorption dans les éléments biochimiques.

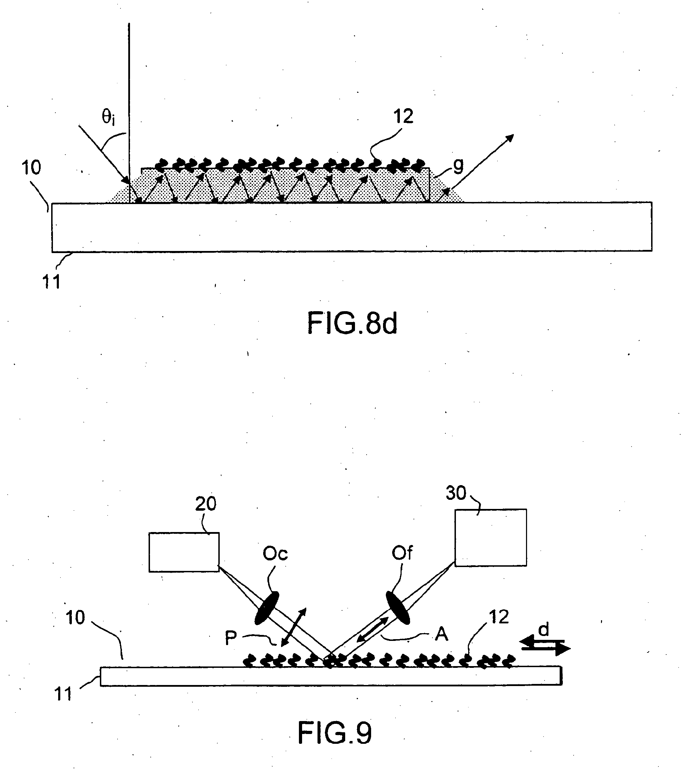

On peut encore prévoir un guide d'onde g avec un indice n' élevé entre la surface de la lame support 11 et les spots d'Adn 12 comme illustré sur la figure 8d, pour favoriser un effet de multipassage de la lumière, obtenu par réflexion totale interne, à l'intérieur du guide d'onde g. De manière connue cette réflexion totale interne est obtenue lorsque l'angle d'incidence θᵢ de la lumière émise par la source sur les surfaces du guide d'onde g ne permet pas d'en ressortir. Une condition nécessaire est que le milieu extérieur soit d'indice plus faible que celui du guide. Il s'agit par exemple de l'air (n∼1) ou de l'eau (n∼1,33). L'onde ne peut pas alors être transmise dans le milieu d'indice plus faible sans respecter la loi de Snell-Descartes. Une réflexion totale interne a également lieu à l'interface entre guide optique et substrat si celui-ci présente un indice plus faible que celui du guide.
Le multipassage de la lumière dans le guide d'onde permet d'amplifier le signal d'absorption. A chaque réflexion sur la paroi supérieure du guide, on a une absorption par les spots d'ADN des ondes évanescentes présentes à l'extérieur du guide, absorption qui peut s'accumuler avec les réflexions successives.
Le multipassage permet aussi d'amplifier un effet de dichroïsme, qui améliore le contraste. Cet effet de dichroïsme est lié à l'exploitation de la polarisation de la lumière. En effet, en éclairant une lame support 11 recouverte de spots d'ADN 12 (biopuce 10) avec une lumière polarisée, le dichroïsme du matériel biologique qui est particulièrement marqué en ultraviolet, permet d'accroître le contraste entre les endroits recouverts d'ADN et le reste de la surface de la lame support. Un polariseur pour la lumière incidente et un analyseur pour la lumière réfléchie permettent d'exploiter ce dichroïsme.

Ainsi, il est possible d'exploiter de manière avantageuse la polarisation de la lumière pour améliorer la détection, par augmentation du contraste entre les endroits recouverts d'ADN et le reste de la surface de la lame support.

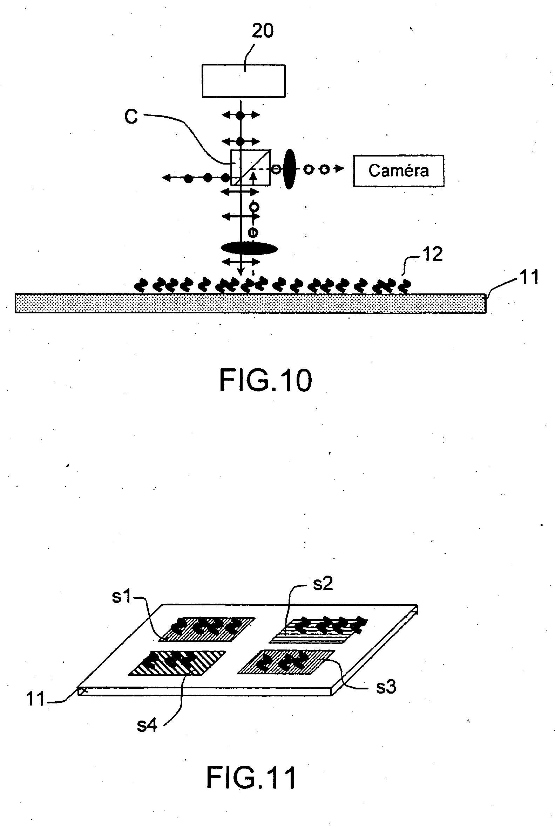

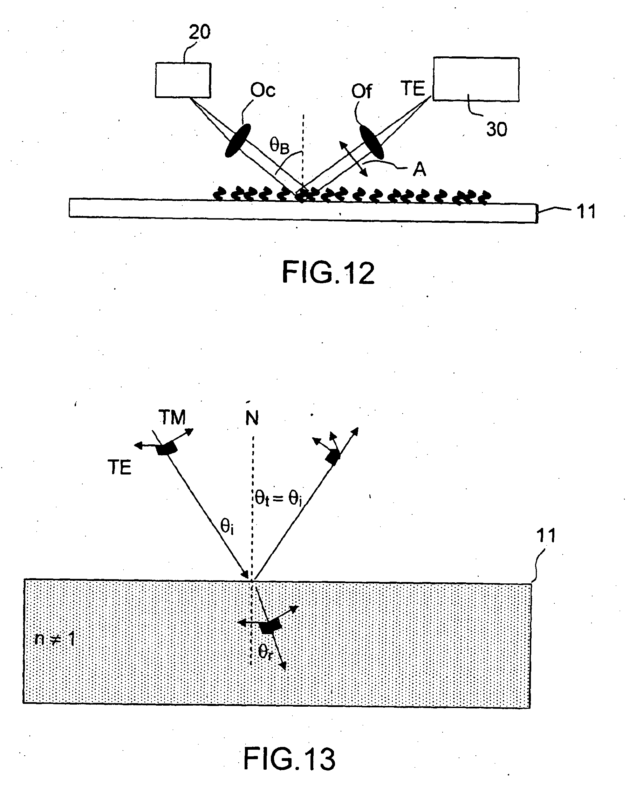

Rappelons brièvement les différents états de polarisation de la lumière avant et après réflexions sur une surface, en référence avec la figure 13 : On appelle polarisation transverse électrique TE la configuration pour laquelle la composante électrique de l'onde électromagnétique est polarisée perpendiculairement au plan d'incidence (le plan contenant les rayons incidents et réfléchis). La composante du champ magnétique associée est alors dans ce plan d'incidence. Inversement, on appelle polarisation transverse magnétique TM la configuration pour laquelle le champ magnétique est perpendiculaire au plan d'incidence et la composante du champ électrique dans le plan d'incidence. On note respectivement θᵢ, θₜ, θᵣ, les angles d'incidence, de réflexion et de réfraction, n l'indice de la lame support 11, n différent de 1 et N la direction de la normale à la surface de la lame.
Les coefficients de réflexion et transmission sont définis pour chaque polarisation et dépendent de l'angle. En éclairant une lame recouverte de spots d'ADN avec une lumière polarisée, le dichroïsme du matériel biologique particulièrement marqué en ultraviolet permet d'accroître le contraste entre les endroits recouverts d'ADN et le reste de la lame. Un polariseur P pour la lumière incidente et un analyseur A pour la lumière réfléchie permettent d'exploiter ce dichroïsme, comme illustré sur la figure 9. Dans un exemple pratique de mise en oeuvre, on trouve alors sur le trajet du faisceau incident entre la source et la surface de la lame support, une optique de collimation Oc et un polariseur P ; et sur le trajet du faisceau réfléchi par la surface de la lame support 11, vers le dispositif de détection, une optique de focalisation Of et un analyseur A (figure 9). Pour couvrir toute la surface, la lame support 10 est déplacée (d) relativement à l'axe de focalisation optique.
Dans une variante illustrée sur la figure 10, on utilise un dispositif séparateur polarisant C en incidence normale. Sur la figure on note par des flèches la polarisation dans le plan de la feuille, et par des points, la polarisation perpendiculaire. Le dispositif C laisse ainsi passer une seule polarisation vers la surface de la lame support, l'autre polarisation étant envoyée dans une autre direction. La polarisation réfléchie, représentée par des cercles est envoyée vers la caméra. Le dispositif permet d'éclairer la lame avec une seule polarisation, et d'envoyer la polarisation réfléchie vers une caméra sur laquelle le dichroïsme est ainsi directement visualisé. Ce dispositif séparateur polarisant C peut être un dispositif classique de séparation de faisceaux polarisés par exemple, un polariseur à prismes Glan-Taylor.
Dans ces deux cas illustrés sur la figure 9 et la figure 10, on pourra utiliser le formalisme de Stokes-Mueller pour décrire l'évolution de la polarisation de la lumière lors de la transmission et la réflexion de la lumière sur la lame support de spot ADN. On rappelle que la matrice de Mueller est une matrice d'ordre 4 qui s'applique au vecteur de Stokes composé de 4 éléments :
- L'intensité : I = <EₓEₓ*+E_{y}E_{y}*>
- La polarisation linéaire : I = <EₓEₓ*-E_{y}E_{y}*>
- La polarisation à 45° : U = <EₓE_{y}*+E_{y}Eₓ*>
- La polarisation circulaire droite : V = <EₓE_{y}*-E_{y}Eₓ*>

Dans un perfectionnement, on réalise sur la surface de la lame support 11 des structurations rectilignes et parallèles s1, s2, s3, s4 analogues à des réseaux de diffraction, comme illustré en exemples sur la figure 11. Ces structurations permettent d'obtenir un meilleur coefficient de réflexion pour des polarisations données, fonction du sens de la structuration. On peut ainsi favoriser ainsi une polarisation de réflexion donnée, et notamment une polarisation circulaire. Comme illustré, on peut prévoir plusieurs structurations différentes (orientations rectilignes différentes) sur la même lame support 10, ce qui favorise des détections différentielles.

Une mise en oeuvre avantageuse de l'utilisation de la polarisation de la lumière permet par rapport au système d'imagerie illustré sur la figure 9, de supprimer le polariseur P, comme illustré sur la figure 12. Dans cette mise en oeuvre, on utilise comme angle d'incidence θᵢ de l'éclairage ultra-violet émis par la source, l'angle dit de Brewster θ_{B}. L'angle de Brewster θ_{B} est l'angle pour lequel la réflexion de la polarisation TM s'annule et la lumière réfléchie est donc exclusivement dans la polarisation TE. Dans cette mise en oeuvre, le dichroïsme du matériel biologique particulièrement marqué en ultraviolet se traduit alors par une polarisation TM plus forte pour les endroits de la surface de la lame support 11 recouverts d'ADN.

## Revendications

1. Système d'imagerie d'éléments biochimiques comprenant une biopuce (10) comprenant un support sensiblement plan (11), présentant sur une face supérieure (11a), une pluralité de spots, sur lesquels sont disposés des éléments biochimiques (12) à analyser, une source (20) pour éclairer ladite face supérieure de la biopuce avec un faisceau lumineux et un dispositif de détection (30) d'un rayonnement émis par la dite face supérieure, **caractérisé en ce que** le dispositif de détection est agencé pour détecter le faisceau lumineux réfléchi ou transmis par ladite face supérieure du support de la biopuce, **en ce que** la source et le dispositif de détection opèrent chacun dans une bande de longueurs d'ondes comprenant une bande spectrale couvrant au moins une longueur d'onde d'intérêt déterminée (λᵢ) comprise dans la bande de rayonnement ultra-violet, **en ce que** l'un des deux au moins de la source et du dispositif de détection est sélectif dans ladite bande spectrale et **en ce que** le support de ladite biopuce présente un coefficient de réflectivité ou de transmission dans ladite bande spectrale dont une limite basse est de l'ordre de 10% permettant un fonctionnement du système en réflexion ou en transmission.

2. Système selon la revendication 1, **caractérisé en ce que** la source et/ou le système de détection opèrent dans une fenêtre de longueur d'onde comprise ou égale à une bande 240-290 nanomètres.

3. Système selon la revendication 2, **caractérisé en ce que** ledit dispositif de détection (51) est un dispositif semi-conducteur à bande spectrale étroite.

4. Système selon la revendication 3, **caractérisé en ce que** la source est une source de lumière blanche (50) disposée pour éclairer la puce par la face arrière dudit dispositif de détection.

5. Système selon la revendication 2, **caractérisé en ce que** la source est une source blanche associée à un filtre de sélection centré sur ladite longueur d'onde d'intérêt (λᵢ).

6. Système selon la revendication 1, **caractérisé en ce qu'**une limite basse du coefficient de réflectivité ou de transmission à ladite longueur d'onde d'intérêt est de l'ordre de 10 pour cent.

7. Système selon l'une quelconque des revendications 1 à 6, **caractérisé en ce que** la face supérieure (11a) de la biopuce (10) présente une texture telle que le signal d'absorption généré par les éléments biochimiques immobilisés sur les spots est amplifié.

8. Système selon la revendication 7, **caractérisé en ce que** la face supérieure (11a) de la biopuce présente une rugosité de surface permettant d'augmenter la surface apparente de la biopuce.

9. Système selon la revendication 7, **caractérisé en ce que** la face supérieure (11) de la biopuce comporte des motifs sur lesquels sont disposés les spots, lesdits motifs étant tels que le chemin optique du faisceau lumineux à travers les éléments biochimiques est augmenté.

10. Système selon la revendication 9, **caractérisé en ce que** lesdits motifs sont des motifs géométriques protubérants (74, 75), en sorte que la densité d'éléments biochimiques sur chaque spot est augmentée.

11. Système selon la revendication 10, **caractérisé en ce que** lesdits motifs comportent un plan incliné (74) ou vertical (75) sur lequel sont fixés lesdits éléments biochimiques, et **en ce que** le faisceau lumineux a un angle d'incidence (α) sur la surface (11) de la biopuce correspondant à l'angle formé par ledit plan des motifs avec ladite surface (11).

12. Système selon la revendication 9, **caractérisé en ce que** lesdits motifs sont des porosités, le support comprenant au moins trois couches, une première couche poreuse (80), dont des porosités forment des spots sur lesquels sont immobilisés des éléments biochimiques, et une couche de part et d'autre (81, 82), l'une au moins également poreuse en sorte de permettre une hybridation, lesdites couches de part et d'autre présentant chacune une face réfléchissante vers ladite première couche poreuse (80), en sorte que le chemin optique est augmenté par effet de réflexion dans ladite première couche poreuse (80).

13. Système selon l'une quelconque des revendications 1 à 8, **caractérisé en ce qu'**un guide d'onde (g) est prévu sur la surface du support (10) de la biopuce, les spots d'éléments biochimiques étant déposés sur la surface supérieure du guide d'onde, ledit guide d'onde permettant un multipassage de la lumière dans le guide d'onde.

14. Système selon l'une quelconque des revendications précédentes, **caractérisé en ce qu'**il comprend des moyens de polarisation de la lumière pour augmenter le contraste entre les zones du support comportant des éléments biochimiques et celles qui n'en comportent pas.

15. Système selon la revendication 14, **caractérisé en ce que** ledit support comprend sur sa face supérieure un ou des réseaux sous forme de structurations rectilignes parallèles (s1), sur lesquels sont déposés des spots d'éléments biochimiques, chaque réseau favorisant une polarisation de réflexion donnée, fonction du sens de la structuration rectiligne correspondante.

## Patentansprüche

1. Abbildungssystem biochemischer Elemente, die einen Biochip (10) umfassen, der einen etwa ebenen Träger (11), der auf einer Oberseite (11a) eine Vielzahl von Spots aufweist, auf denen zu analysierende biochemische Elemente (12) angeordnet sind, eine Quelle (20), um die Oberseite des Biochips mit einem Lichtstrahl zu beleuchten und eine Erkennungsvorrichtung (30) einer von der Oberseite gesendeten Strahlung umfasst, **dadurch gekennzeichnet, dass** die Erkennungsvorrichtung ausgebildet ist, um den von der Oberseite des Trägers des Biochips reflektierten oder übertragenen Lichtstrahl zu erkennen, dass die Quelle und die Erkennungsvorrichtung jeweils in einem Wellenlängenbereich arbeiten, der ein Spektralband umfasst, das mindestens eine bestimmte interessierende Wellenlänge (λᵢ) abdeckt, die sich im ultravioletten Strahlungsbereich befindet, dass mindestens entweder die Quelle oder die Erkennungsvorrichtung im Spektralband selektiv ist und dass der Träger des Biochips einen Reflektivitäts- oder Übertragungskoeffizienten in dem Spektralband aufweist, von dem ein unterer Grenzwert zirka 10 % beträgt, wodurch eine reflektierende oder übertragende Funktionsweise des Systems möglich ist.

2. System nach Anspruch 1, **dadurch gekennzeichnet, dass** die Quelle und/oder das Erkennungssystem in einem Wellenlängenfenster arbeiten, das ein Band 240-290 Nanometer umfasst oder diesem entspricht.

3. System nach Anspruch 2, **dadurch gekennzeichnet, dass** die Erkennungsvorrichtung (51) eine Halbleitervorrichtung mit engem Spektralband ist.

4. System nach Anspruch 3, **dadurch gekennzeichnet, dass** die Quelle eine Quelle weißen Lichts (50) ist, die angeordnet ist, um den Chip von der Rückseite der Erkennungsvorrichtung zu beleuchten.

5. System nach Anspruch 2, **dadurch gekennzeichnet, dass** die Quelle eine weiße Quelle ist, die einem Auswahlfilter zugeordnet ist, der auf die interessierende Wellenlänge (λᵢ) zentriert ist.

6. System nach Anspruch 1, **dadurch gekennzeichnet, dass** ein unterer Grenzwert des Reflektivitäts- oder Übertragungskoeffizienten zu der interessierenden Wellenlänge zirka 10 Prozent beträgt.

7. System nach einem der Ansprüche 1 bis 6, **dadurch gekennzeichnet, dass** die Oberseite (11a) des Biochips (10) eine Textur aufweist, die derart ist, dass das Absorptionssignal, das von den auf den Spots blockierten biochemischen Elementen erzeugt wird, verstärkt wird.

8. System nach Anspruch 7, **dadurch gekennzeichnet, dass** die Oberseite (11a) des Biochips eine Oberflächenrauheit aufweist, die erlaubt, die sichtbare Fläche des Biochips zu vergrößern.

9. System nach Anspruch 7, **dadurch gekennzeichnet, dass** die Oberseite (11) des Biochips Motive aufweist, auf denen die Spots angeordnet sind, wobei die Motive derart sind, dass der optische Weg des Lichtstrahls durch die biochemischen Elemente verlängert wird.

10. System nach Anspruch 9, **dadurch gekennzeichnet, dass** die Motive vorspringende geometrische Motive (74, 75) sind, so dass die Dichte biochemischer Elemente auf jedem Spot vergrößert ist.

11. System nach Anspruch 10, **dadurch gekennzeichnet, dass** die Motive eine geneigte (74) oder vertikale (75) Ebene aufweisen, auf der die biochemischen Elemente fixiert sind, und dass der Lichtstrahl einen Einfallswinkel (α) auf die Fläche (11) des Biochips hat, der dem Winkel entspricht, der von der Ebene der Motive mit der Fläche (11) gebildet wird.

12. System nach Anspruch 9, **dadurch gekennzeichnet, dass** die Motive Porositäten sind, wobei der Träger mindestens drei Schichten umfasst, eine erste poröse Schicht (80), deren Porositäten Spots bilden, auf denen biochemische Elemente blockiert sind, und eine Schicht auf der einen und der anderen Seite (81, 82), wobei eine mindestens ebenfalls derart porös ist, dass eine Hybridation möglich ist, wobei die Schichten auf der einen und der anderen Seite jeweils eine zur ersten porösen Schicht (80) reflektierende Seite aufweisen, so dass der optische Weg durch Reflexionseffekt in der ersten porösen Schicht (80) verlängert wird.

13. System nach einem der Ansprüche 1 bis 8, **dadurch gekennzeichnet, dass** eine Wellenführung (g) auf der Fläche des Trägers (10) des Biochips vorgesehen ist, wobei die Spots biochemischer Elemente auf der Oberseite der Wellenfuhrung angeordnet sind, wobei die Wellenführung eine Multipassage des Lichts in der Wellenführung erlaubt.

14. System nach einem der vorangehenden Ansprüche, **dadurch gekennzeichnet, dass** es Polarisationsmittel des Lichts umfasst, um den Kontrast zwischen den Zonen des Trägers zu erhöhen, die biochemische Elemente aufweisen und denen, die diese nicht aufweisen.

15. System nach Anspruch 14, **dadurch gekennzeichnet, dass** der Träger auf seiner Oberseite ein Netzwerk oder Netzwerke in Form paralleler geradliniger Strukturierungen (s1) umfasst, auf denen Spots biochemischer Elemente aufgebracht sind, wobei jedes Netzwerk eine bestimmte Reflexionspolarisierung fördert, die von der Richtung der entsprechenden geradlinigen Strukturierung abhängt.

## Claims

1. Imagery system for biochemical elements comprising a biochip (10) comprising an approximately plane support (11), with a plurality of spots on an upper face (11a), on which biochemical elements (12) to be analysed are arranged, a source (20) to illuminate with a light beam said upper face of the biochip and a device (30) for detection of radiation emitted by said upper face, **characterised in that** the detection device is arranged to detect the light beam which is reflected or transmitted by said upper face of the biochip's support, **in that** the source and the detection device each function in a wavelength band comprising a spectral band covering at least a determined wavelength of interest (λᵢ) within the ultraviolet radiation band, **in that** at least either the source or the detection device is selective at said spectral band and **in that** the support of said biochip has a reflectivity or transmission coefficient in said spectral band for which the lower limit is of the order of 10 percent such that the system will operate in reflection or in transmission.

2. System according to claim 1, **characterised in that** the source and/or the detection device function in a window with a wavelength inside or equal to a 240-290 nanometres band.

3. System according to claim 2, **characterised in that** said detection device (51) is a semiconducting device with a narrow spectral band.

4. System according to claim 3, **characterised in that** the source is a white light source (50) arranged to illuminate the chip from the back face of said detection device.

5. System according to claim 2, **characterised in that** the source is a white source associated with a selection filter centred on said wavelength of interest (λᵢ).

6. System according to claim 1, **characterised in that** the lower limit of the reflectivity or transmission coefficient at said wavelength of interest (λᵢ) is of the order of 10 percent.

7. System according to any one of claims 1 to 6, **characterised in that** the upper face (11a) of the biochip (10) has a texture such that the absorption signal generated by the biochemical elements immobilised on the spots is amplified.

8. System according to claim 7, **characterised in that** the upper face (11 a) of the biochip has a rough surface making it possible to increase the visible surface of the biochip.

9. System according to claim 7, **characterised in that** the upper face (11a) of the biochip comprises patterns on which spots are arranged, said patterns being such that the optical path of the light beam through the biochemical elements is lengthened.

10. System according to claim 9, **characterised in that** said patterns are protuberant geometric patterns (74, 75), such that the density of biochemical elements on each spot is increased.

11. System according to claim 10, **characterised in that** said patterns comprise an inclined (74) or vertical (75) plane onto which said biochemical elements are grafted, and **in that** the light beam has an incidence angle (α) on the surface (11) of the biochip corresponding to the angle formed by said plane of the patterns with said surface (11).

12. System according to claim 9, **characterised in that** said patterns are porosities, the support comprising at least three layers, a first porous layer (80) for which the porosities form spots on which biochemical elements are immobilised, and a layer on each side (81, 82), at least one of which is also porous so as to make hybridization possible, said layers on each side having a reflecting face towards said first porous layer (80), such that the optical path is enhanced by the reflection effect in said first porous layer (80).

13. System according to any one of claims 1 to 8, **characterised in that** a waveguide (g) is provided on the surface of the support (10) of the biochip, the spots of biochemical elements being deposited on the upper face of the waveguide, said waveguide enabling multi-passes of light in the waveguide.

14. System according to any one of the previous claims, **characterised in that** it comprises means of polarising light to increase the contrast between zones of the support comprising biochemical elements and zones with no biochemical elements.

15. System according to claim 14, **characterised in that** said support comprises one or more gratings in the form of straight parallel structures (s1) on its upper face, on which spots of biochemical elements are deposited, each grating facilitating a given reflection polarisation, depending on the direction of the corresponding straight structure.
